# EUROPÄISCHE PATENTANMELDUNG

(11) **EP 2 811 452 A1**
(43) Veröffentlichungstag der Anmeldung: **10.12.2014**
(21) Anmeldenummer: 13187999.1
(22) Anmeldetag: 10.10.2013
(51) Int. Cl.: G06Q 50/22, G09B 5/06

(54) **Informationssystem**

(30) Priorität: 07.06.2013 DE 202013102445 U
(71) Anmelder: Eckardt, Manuel, 35781 Weilburg (DE)
(72) Erfinder: Eckardt, Manuel, 35781 Weilburg (DE)
(74) Vertreter: Ruckh, Rainer Gerhard

(57) **Zusammenfassung**

Die Erfindung betrifft ein Informationssystem mit einer Rechnereinheit, auf welcher Informationen betreffend den Bereich eines Fitness-Studios gespeichert sind. Die Informationen sind über eine Schnittstelle der Rechnereinheit eingebbar, wobei die Rechnereinheit eine tragbare Einheit bildet und von einem Benutzer während des Besuchs eines Fitness-Studios mitgeführt wird. Auf der Rechnereinheit ist ein Auswahlmenü vorgesehen, über welches der Benutzer Informationen seiner Wahl abrufen kann.

## Beschreibung

Die Erfindung betrifft ein Informationssystem.

Dieses Informationssystem wird im Bereich von Fitness-Studios eingesetzt, um einem Benutzer, das heißt einem Besucher des Fitness-Studios, Informationen, die auf das Fitness-Studio und die von diesen erbrachten Dienstleistungen bezogen sind, zur Verfügung zu stellen.

Für Besucher eines Fitness-Studios besteht ein häufig auftretendes Problem darin, dass die Betreuung unzureichend ist. Für die Betreuung eines Benutzers steht zwar generell das Personal des Fitness-Studios zur Verfügung. Problematisch hierbei ist jedoch, dass insbesondere zu den Hauptnutzungszeiten, wie nach Feierabend, die Fitness-Studios sehr stark frequentiert sind, das heißt es sind sehr viele Benutzer im Fitness-Studio, die die dort vorhandenen Einrichtungen gleichzeitig nutzen möchten. Benötigt ein Benutzer dann einen spezifischen Rat zur Durchführung von Übungen oder auch eine allgemeine Information zu dem Bereich Fitness, so steht das Personal des Fitness-Studios infolge von Überlastung hierzu nicht oder nur mit unerwünscht großen Wartezeiten zur Verfügung.

Ein weiterer wesentlicher Nachteil besteht darin, dass der Ausbildungsstand des Personals in Fitness-Studios stark unterschiedlich ist. So kommt es oft vor, dass nur unzureichend geschultes Personal vorhanden ist, welches den Benutzer nur unzureichend beraten kann.

Der Erfindung liegt die Aufgabe zugrunde, ein Informationssystem bereitzustellen, welches einem Benutzer eine verbesserte Nutzung eines Fitness-Studios ermöglicht.

Zur Lösung dieser Aufgabe sind die Merkmale des Anspruchs 1 vorgesehen. Vorteilhafte Ausführungsformen und zweckmäßige Weiterbildungen der Erfindung sind in den Unteransprüchen beschrieben.

Das erfindungsgemäße Informationssystem umfasst eine Rechnereinheit, auf welcher Informationen betreffend den Bereich eines Fitness-Studios gespeichert sind. Die Informationen sind über eine Schnittstelle der Rechnereinheit eingebbar, wobei die Rechnereinheit eine tragbare Einheit bildet und von einem Benutzer während des Besuchs eines Fitness-Studios mitgeführt wird. Auf der Rechnereinheit ist ein Auswahlmenü vorgesehen, über welches der Benutzer Informationen seiner Wahl abrufen kann.

Der Grundgedanke der Erfindung besteht somit darin, ein Informationssystem auf einer tragbaren Rechnereinheit bereitzustellen, welche von einem Benutzer während des Besuchs eines Fitness-Studios mitgeführt werden kann.

Das Informationssystem enthält Information, die für die Nutzung des Fitness-Studios hilfreich oder allgemein von Interesse sind. Das Informationssystem bietet somit dem Benutzer eine unmittelbare Hilfe und Informationsquelle bei der Nutzung des Fitness-Studios, wobei insbesondere die im Informationssystem vorhandenen Informationen Fragen hinsichtlich der Nutzung des Fitness-Studios beantworten. Durch dieses Informationssystem ist der Benutzer nicht mehr oder nur noch in geringem Umfang von der Beratung des Personals im Fitness-Studio abhängig. Dies bedeutet einerseits für den Benutzer selbst einen erheblichen Vorteil, da er nicht darauf angewiesen ist, ob im Fitness-Studio genügend Personal oder ausreichend qualifiziertes Personal zur Beantwortung seiner Fragen vorhanden ist. Andererseits wird durch das Informationssystem auch das Personal des Fitness-Studios selbst entlastet.

Besonders vorteilhaft ist die tragbare Rechnereinheit von einem Tablett-PC gebildet. Dieser Tablett-PC bildet eine handliche Einheit welche der Benutzer bei dem Besuch des Fitness-Studios bequem mitführen kann. Prinzipiell kann die Rechnereinheit auch von einem Smartphone gebildet sein. Dieses ist noch handlicher als ein Tablett-PC, jedoch ist die Bildschirmgröße signifikant kleiner, so dass die Informationen auf dem Tablett-PC besser sichtbar sind als auf dem Smartphone.

Die Rechnereinheit bildet generell eine autarke Einheit, das heißt während der Benutzer die Rechnereinheit, insbesondere den Tablett-PC, im Fitness-Studio mit sich führt, ist diese Rechnereinheit für sich alleine voll funktionsfähig. Damit muss der Benutzer die Rechnereinheit nicht an andere Einheiten anschließen um diese nutzen zu können. Die so ausgebildete Rechnereinheit bietet daher dem Benutzer einen hohen Bedienkomfort.

Insbesondere zum Aktualisieren der auf der Rechnereinheit gespeicherten Informationen weist die Rechnereinheit eine Schnittstelle, die vorteilhaft einen Internet-Anschluss gebildet ist, auf. Besonders zweckmäßig ist dabei als berührungslos arbeitender Internet-Anschluss eine WLAN-Verbindung vorgesehen. Bei modernen Tablett-PCs oder Smartphones können auch über eine sogenannte Cloud mehrere Einheiten über das Internet zusammengeschlossen werden, um so Daten zwischen diesen Einheiten auszutauschen.

Gemäß einer vorteilhaften Ausgestaltung der Erfindung wird über den Internet-Anschluss ein Zugang zu einer externen Einheit hergestellt, auf welcher persönliche Daten des Benutzers vorhanden sind.

Über den Internet-Anschluss kann der Benutzer insbesondere über eine WLAN-Verbindung oder auch mittels einer Cloud auf persönliche Daten zugreifen, die auf entfernten externen Rechnern hinterlegt sind.

Gemäß einer vorteilhaften Ausgestaltung sind als Informationen Videos auf der Rechnereinheit gespeichert.

Diese Videos können als standardisierte Videos Informationen über die Bereiche Fitness, Ernährung und/oder Gesundheit enthalten, das heißt Informationen die direkt mit dem Umfeld eines Fitness-Studios verknüpft sind.

Zusätzlich können auch auf der Rechnereinheit personalisierte Informationen gespeichert sein.

Für den Benutzer besteht dabei die Möglichkeit, über das Auswahlmenü der Rechnereinheit Videos nach seiner Wahl zu bestellen und auf seiner Rechnereinheit zu speichern.

Generell können als personalisierte Informationen Trainingspläne für die Nutzung des Fitness-Studios auf der Rechnereinheit gespeichert werden. Diese Trainingspläne können vom Personal des Fitness-Studios erstellt und auf die Rechnereinheit des Benutzers überspielt werden. Generell kann der Benutzer die Trainingspläne auch selbst erweitern oder mit spezifischen Zusatzinformationen, insbesondere mit seinen eigenen Leistungsdaten, ergänzen.

Die Erfindung wird im Folgenden anhand der Zeichnungen erläutert. Es zeigen:
- Figur 1:: Schematische Darstellung eines Tablett-PC als Bestandteil des erfindungsgemäßen Informationssystems.
- Figur 2:: Blockschaltbild mit Komponenten des Tablett-PCs gemäß Figur 1.
- Figur 3:: Beispiel eines Auswahlmenüs für den Tablett-PC gemäß den Figuren 1 und 2.
- Figur 4:: Beispiel eines Auswahlmenüs zur Bestellung eines Films für den Tablett-PC gemäß den Figuren 1 und 2.

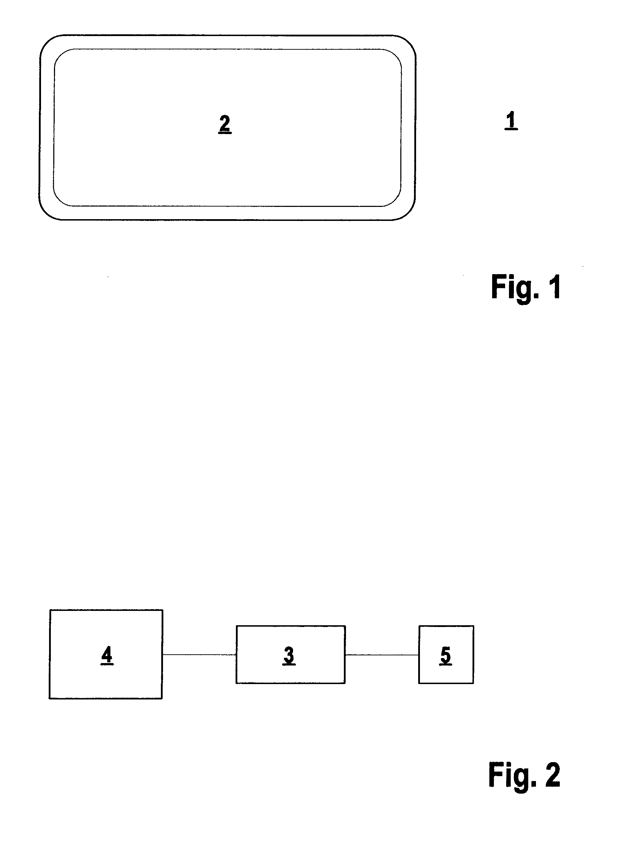

Figur 1 zeigt schematisch als Ausführungsbeispiel einer Rechnereinheit für das erfindungsgemäße Informationssystem einen Tablett-PC 1. Figur 2 zeigt in stark vereinfachter Form ein Blockschaltbild mit den wesentlichen Komponenten des Tablett-PCs 1.

Der Tablett-PC 1 weist, wie in Figur 1 schematisch dargestellt, in bekannter Weise einen Bildschirm 2 mit Touch-Screen-Funktion auf.

Wie Figur 2 zeigt, weist der Tablett-PC 1 ein Prozessorsystem 3 mit einer Anzahl von Mikroprozessoren auf, welche die Funktionen des Tablett-PCs 1 steuern. Weiterhin sind Ein-/ Ausgabeeinheiten 4 mit dem Bildschirm 2 und den Touch-Screen-Funktionen vorgesehen. Schließlich ist eine Schnittstelleneinheit 5 vorgesehen, die unterschiedliche Schnittstellen aufweist. Insbesondere ist als Schnittstelle ein Internet-Anschluss vorgesehen. Der Internet-Anschluss umfasst einen WLAN-Anschluss und eine Cloud, mit der der Tablett-PC 1 mit weiteren externen Einheiten kommunizieren kann.

Der Tablett-PC 1 bildet eine tragbare Rechnereinheit, die von einem Benutzer, das heißt einem Besucher eines Fitness-Studios, während seines Aufenthalts im Fitness-Studio mitgeführt werden kann, um für das Fitness-Studio beziehungsweise das dort durchzuführende Training relevante Informationen abzurufen.

In seiner einfachsten Ausgestaltung wird der Tablett-PC 1 als autarke Rechnereinheit genutzt, das heißt es werden keine Verbindungen des Tablett-PC 1 zu externen Einheiten benötigt. Der Benutzer nutzt dann allein auf dem Tablett-PC 1 abgespeicherte, standardisierte Informationen, die er über ein Auswahlmenü des Tablett-PCs 1 auswählen und abrufen kann.

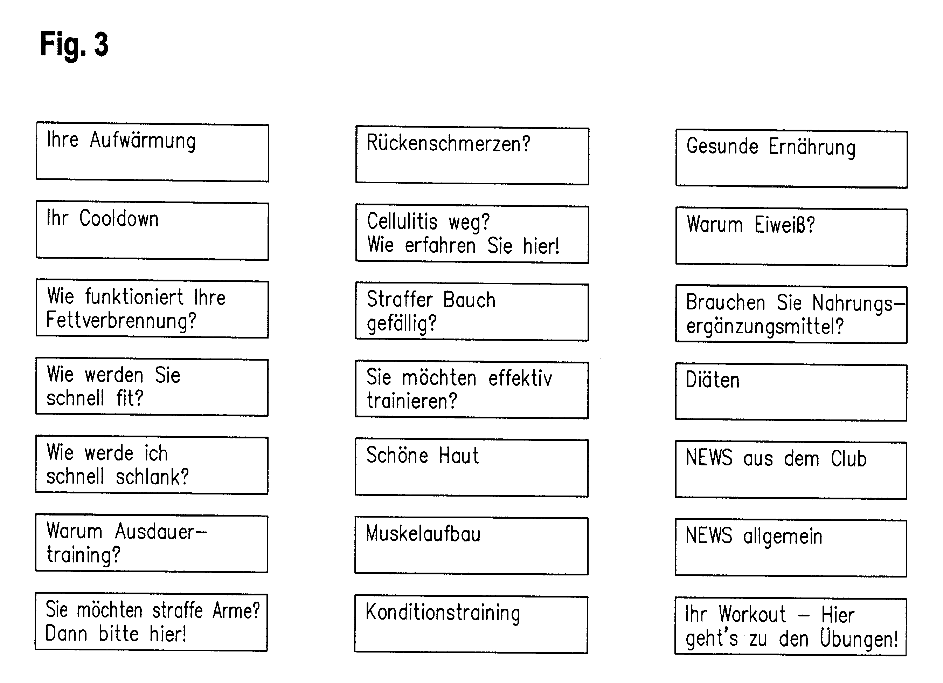

Figur 3 zeigt ein Ausführungsbeispiel eines solchen Auswahlmenüs. Durch Andrücken einzelner Buttons des Auswahlmenüs kann der Benutzer Videos, das heißt Filme abrufen und während seines Besuchs im Fitness-Studio abspielen lassen.

Die Videos befassen sich generell mit für das Fitness-Studio relevanten Themen, insbesondere Fitness-Themen, aber auch verwandte Themen wie Gesundheit und Ernährung.

Beispielsweise kann der Benutzer durch Berühren des Buttons "Ihre Aufwärmung" einen Film auswählen und auf seinem Tablett-PC 1 abspielen lassen, der dem Benutzer unterschiedliche Übungen und Informationen über ein gezieltes Aufwärmen zeigt.

Entsprechendes gilt für Entspannungsübungen nach dem Training. Einen Film hierzu kann der Benutzer über den Button "Ihr Cooldown" abrufen.

Weitere Filme betreffen das Thema Gesundheit, wie zum Beispiel die zu den Buttons "Wie werde ich schnell schlank", "Schöne Haut" hinterlegten Video.

Weitere Filme betreffen das Thema Ernährung, wie zum Beispiel die zu den Buttons "Diäten" "Warum Eiweiß" hinterlegten Video.

Schließlich sind auch Filme vorhanden, die gezielt das Durchführen des Trainings im Fitness-Studio betreffen, wie zum Beispiel das zum Button "Ihr Workout - Hier geht's zu den Übungen" hinterlegte Video.

Gemäß einer ersten Ausbaustufe ist der Tablett-PC 1 über die Schnittstelle, insbesondere den Internet-Anschluss, an externe Einheiten angeschlossen, damit die auf dem Tablett-PC 1 gespeicherten Filme aktualisiert werden können. Hierzu werden beispielsweise vom Betreiber des Fitness-Studios aktuelle Filme auf den Tablett-PC 1 geladen. Der Betreiber kann die Internetverbindung auch dazu nutzten, den Benutzern aktuelle Meldungen betreffend das Fitness-Studio, wie geänderte Öffnungszeiten, das Angebot neuer Kurse und dergleichen, auf den Tablett-PC 1 zu spielen.

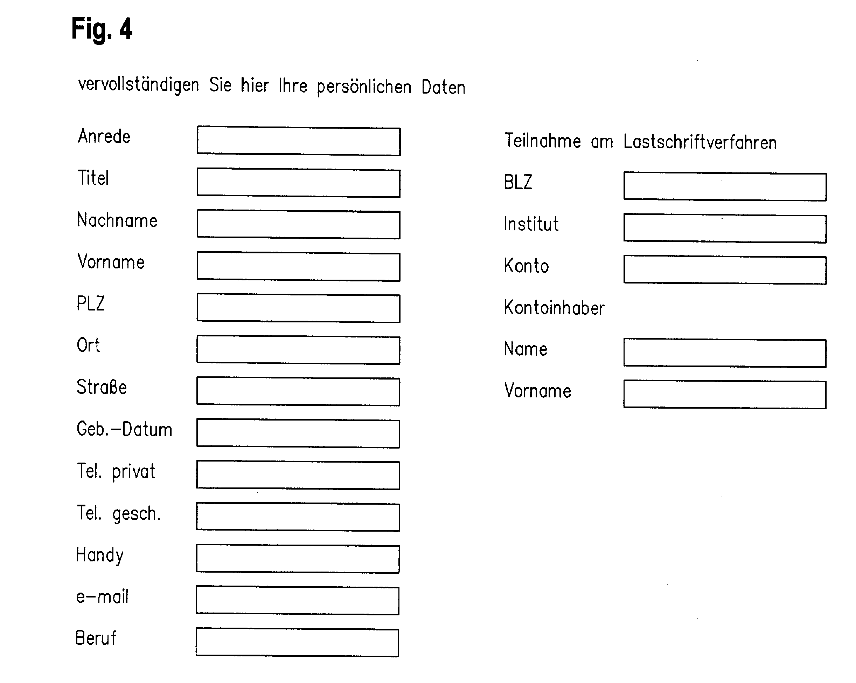

Gemäß einer weiteren Ausbaustufe kann der Benutzer den Internet-Anschluss seines Tablett-PC 1 auch dazu nutzen, um selbst individuelle Filme zu bestellen. Figur 4 zeigt beispielhaft ein Auswahlmenü hierfür.

Ein entsprechendes Auswahlmenü kann auch vorgesehen sein, damit ein Benutzer sich bei dem Fitness-Studio für bestimmte Trainingseinheiten oder allgemein als Mitglied anmeldet.

Gemäß einer letzen Ausbaustufe kann der Tablett-PC 1 auch zur Nutzung personalisierter Daten dienen.

Beispielweise kann der Benutzer des Tablett-PCs 1 über eine Intemetverbindung, insbesondere über eine sogenannte Cloud, einen Zugang zu einer externen Rechnereinheit, die von ihm genutzt wird, herstellen, um so einen Zugriff auf seine persönlichen Daten während des Besuchs des Fitness-Studios zu erhalten.

Weiterhin können auf dem Tablett-PC 1 auch personalisierte Daten in Form von Trainingsplänen gespeichert sein. Die Trainingspläne werden zum Beispiel bei einem Einführungstraining mit dem Personal des Fitness-Studios definiert und festgelegt. Der Betreiber stellt dann den individuellen Trainingsplan dem Benutzer zur Verfügung, indem er diesen auf den Tablett-PC 1 des Benutzers lädt. Der Trainingsplan kann dann fortlaufend aktualisiert werden. Auch können aktuelle Leistungsdaten zu dem Trainingsplan hinterlegt und vom Tablett-PC 1 abgerufen werden.

### Bezugszeichenliste

(1) Tablett-PC
(2) Bildschirm
(3) Prozessorsystem
(4) Ein-/ Ausgabeeinheit
(5) Schnittstelleneinheit

## Patentansprüche

1. Informationssystem mit einer Rechnereinheit, auf welcher Informationen betreffend den Bereich eines Fitness-Studios gespeichert sind, wobei die Informationen über eine Schnittstelle der Rechnereinheit eingebbar sind, wobei die Rechnereinheit eine tragbare Einheit bildet und von einem Benutzer während des Besuchs eines Fitness-Studios mitgeführt wird, und wobei auf der Rechnereinheit ein Auswahlmenü vorgesehen ist, über welches der Benutzer Informationen seiner Wahl abrufen kann.

2. Informationssystem nach Anspruch 1, **dadurch gekennzeichnet, dass** die Rechnereinheit ein Tablett-PC (1) oder ein Smart-Phone ist.

3. Informationssystem nach einem der Ansprüche 1 oder 2, **dadurch gekennzeichnet, dass** als Schnittstelle ein Internet-Anschluss vorgesehen ist.

4. Informationssystem nach Anspruch 3, **dadurch gekennzeichnet, dass** als Schnittstelle ein drahtloser Internet-Anschluss vorgesehen ist.

5. Informationssystem nach Anspruch 4, **dadurch gekennzeichnet, dass** über die Schnittstelle Informationen auf der Rechnereinheit aktualisierbar sind.

6. Informationssystem nach Anspruch 5, **dadurch gekennzeichnet, dass** über den Internet-Anschluss ein Zugang zu einer externen Einheit hergestellt wird, auf welcher persönliche Daten des Benutzers vorhanden sind.

7. Informationssystem nach einem der Ansprüche 1 bis 6, **dadurch gekennzeichnet, dass** als Informationen Videos auf der Rechnereinheit gespeichert sind.

8. Informationssystem nach einem der Ansprüche 1 bis 7, **dadurch gekennzeichnet, dass** die Informationen die Bereiche Fitness, Gesundheit und/oder Ernährung betreffen.

9. Informationssystem nach einem der Ansprüche 7 oder 8, **dadurch gekennzeichnet, dass** als Informationen standardisierte Videos auf der Rechnereinheit gespeichert sind.

10. Informationssystem nach einem der Ansprüche 1 bis 9, **dadurch gekennzeichnet, dass** auf der Rechnereinheit personalisierte Informationen gespeichert sind.

11. Informationssystem nach Anspruch 10, **dadurch gekennzeichnet, dass** die personalisierten Informationen von Trainingsplänen des Benutzers gebildet sind.

12. Informationssystem nach Anspruch 10, **dadurch gekennzeichnet, dass** die personalisierten Informationen von Videos gebildet sind, welche vom Benutzer ausgewählt werden.
